Europäisches Patentamt

European Patent Office

Office européen des brevets

(19)

(11)  **EP 0 794 832 B1**

(12)  **EUROPÄISCHE PATENTSCHRIFT**

(45)  Veröffentlichungstag und Bekanntmachung des Hinweises auf die Patenterteilung:
      **12.09.2001  Patentblatt 2001/37**

(51)  Int Cl.[7]: **B01F 17/00**

(21)  Anmeldenummer: **95941008.5**

(86)  Internationale Anmeldenummer:
      **PCT/EP95/04628**

(22)  Anmeldetag: **23.11.1995**

(87)  Internationale Veröffentlichungsnummer:
      **WO 96/16728 (06.06.1996 Gazette 1996/26)**

(54)  **WASSER-IN-ÖL-EMULSIONEN**

WATER-IN-OIL EMULSIONS

EMULSION D'EAU DANS L'HUILE

(84)  Benannte Vertragsstaaten:
      **DE ES FR IT**

(30)  Priorität: **02.12.1994  DE 4442966**

(43)  Veröffentlichungstag der Anmeldung:
      **17.09.1997  Patentblatt 1997/38**

(73)  Patentinhaber: **Henkel Kommanditgesellschaft auf Aktien**
      **40191 Düsseldorf (DE)**

(72)  Erfinder:
      • **ANSMANN, Achim**
        **D-40699 Erkrath (DE)**
      • **CONRADI, Joachim**
        **D-40589 Düsseldorf (DE)**
      • **KAWA, Rolf**
        **D-40789 Monheim (DE)**
      • **SCHIEFERSTEIN, Ludwig**
        **D-40882 Ratingen (DE)**

(56)  Entgegenhaltungen:
      **EP-A- 0 000 424**      **WO-A-93/23450**
      **DE-A- 2 430 342**      **DE-A- 4 202 065**
      **DE-A- 4 315 172**      **GB-A- 2 117 398**

Bemerkungen:
      Die Akte enthält technische Angaben, die nach dem Eingang der Anmeldung eingereicht wurden und die nicht in dieser Patentschrift enthalten sind.

**Beschreibung**

**Gebiet der Erfindung**

Die Erfindung betrifft die Verwendung von Block-Copolyestem als Stabilisatoren zur Herstellung von W/O-Emulsionen, ein Verfahren zur Herstellung der Emulsionen sowie die Emulsionen als solche.

**Stand der Technik**

Die Verwendung von Block-Copolyestem aus Hydroxyfettsäure-Oligomeren und Polyethylenglycolen als Tenside und Emulgatoren ist aus verschiedenen Druckschriften bekannt. So werden in **DE-A-2430342** solche Copolyester als Dispergierhilfsmittel für Kohlenwasserstofföle beschrieben. Aus **EP-B-0000424** sind Block-Copolymere aus 12-Hydroxystearinsäure-Oligomeren und Polyethylenglycolen als Co-Emulgatoren zur Emulgierung von Dieselölen beschrieben. Es zeigte sich aber, daß die dort beschriebenen Block-Copolymeren, die 30 - 62 Gew.-% Polyethylenglycol-Einheiten enthielten, für die Emulgierung von Wasser in polaren Ölkomponenten wenig geeignet sind. In **DE-A-4315172** wird vorgeschlagen, zur Emulgierung von Wasser in Triglyceridöle eine Kombination der aus EP-B-000424 bekannten Block-Copolymeren mit anderen W/O-Emulgatoren mit einem HLB-Wert von 3 bis 7 einzusetzen.

**Beschreibung der Erfindung**

Gegenstand der Erfindung ist die Verwendung von Block-Copolyestem der allgemeinen Formel **(I)**,

$$R^1CO\text{-}(OR^2CO)_y\text{-}(OC_2H_4)_x\text{-}O\text{-}(COR^2O)_z\text{-}COR^1 \qquad (I)$$

in der $R^1CO$ eine Acylgruppe mit 1 bis 22 Kohlenstoffatomen oder eine Hydroxyfettacylgruppe mit 16 bis 22 Kohlenstoffatomen, die Gruppe $(OR^2CO)$ der Rest einer Hydroxyfettacylgruppe mit 16 bis 22 Kohlenstoffatomen, x eine Zahl von 5 bis 150, y und z Zahlen von 2 bis 75 sind, wobei die Gruppe $(OC_2H_4)_x$ wenigstens 10, aber weniger als 20 Gew.-% des Molekulargewichtes ausmacht, zur Stabilisierung von Wasser-in-Öl-Emulsionen.

Überraschend wurde festgestellt, daß durch Erniedrigung des Anteils der hydrophilen Gruppe solcher Block-Copolyester eine Verbesserung der Wasser-in-Öl-Emulgiereigenschaften erzielt werden kann, die sich insbesondere für polare Ölkomponenten günstig auswirkt.

Block-Copolyester

Die Herstellung der Block-Copolyester ist an sich literaturbekannt. Sie kann z.B. dadurch erfolgen, daß man zunächst den lipophilen Molekülteil durch Polykondensation einer Hydroxyfettsäure herstellt. Dabei kann man entweder eine nicht hydroxylierte Carbonsäure der Formel $R^1COOH$ als Startmolekül mit y bzw. z Mol einer Hydroxyfettsäure kondensieren. Man kann aber auch einfach die Hydroxyfettsäure der Formel $HO\text{-}R^2\text{-}COOH$ polykondensieren; in diesem Falle dient die Hydroxyfettsäure selbst als Startmolekül (d.h. $R^1 = HOR^2$). Als Hydroxyfettsäuren eignen sich z. B. die 12-Hydroxystearinsäure und andere synthetische Hydroxyfettsäuren, die z.B. durch Hydrierung von Epoxyfettsäuren mit 16 bis 22 Kohlenstoffatomen zugänglich sind. Eine besonders bevorzugte Hydroxyfettsäure ist die Ricinolsäure und die gehärtete Ricinusölfettsäure. Die Polykondensation wird bevorzugt in Gegenwart eines sauren Katalysators (z.B. Methansulfonsäure) unter Wasserabspaltung in Gegenwart von Toluol oder Xylol als Schleppmittel solange durchgeführt, bis die Wasserabscheidung und die Senkung der Säurezahl einen mittleren Kondensationsgrad (y bzw. z) von 2 bis 75, bevorzugt von 5 bis 30, anzeigen. Bevorzugt werden daher solche Block-Copolyester der Formel **(I)** verwendet, in welchen die Gruppen $(OR^2CO)_y$ und $(COR^2O)_z$ Reste der Ricinolsäure oder der hydrierten Ricinusölfettsäure mit einem Kondensationsgrad y oder z von 5 bis 30 sind. Der hydrophile Molekülteil mit der Formel $(OC_2H_4)_x$ besteht aus einem Polyethylenglycol mit einem mittleren Polymerisationsgrad von x = 5 bis 150, bevorzugt von 10 bis 70. Solche Polyethylenglycole sind im Handel erhältlich. Die Herstellung der Block-Copolyester kann durch Kondensation des lipophilen Hydroxyfettsäure-Polykondensats mit dem Polyethylenglycol unter Abspaltung von 2 Mol Wasser pro Mol Polyethylenglycol erfolgen. Man kann aber auch die Kondensation der Hydroxyfettsäure mit sich selbst und mit dem Polyethylenglycol in einem Reaktionsgemisch in einer einzigen Stufe durchführen. Dabei ist ein Mol Wasser pro Mol Hydroxyfettsäure abzuspalten und eine Säurezahl von weniger als 5 (mg KOH/g) in dem Block-Copolyester anzustreben. Die Herstellung der erfindungsgemäß geeigneten Block-Copolyester läßt sich daher am einfachsten durch Kondensation von 10 bis 20 Gew.-% eines Polyethylenglycols mit 80 bis 90 Gew.-% einer Ricinolsäure oder hydrierter Ricinusölfettsäure unter Abspaltung von 1 Mol Wasser pro Mol Hydroxyfettsäure durchführen. Der HLB-Wert dieser Block-Copolyester, berechnet nach der Formel

$$HLB = \frac{100 - L}{5}$$

(wobei L der Anteil an lipophilen Acylgruppen ($R^1CO$ bzw. $R^2CO$) in Gew.-% ist), liegt daher bei ca. 2 bis 4.

Polare Ölkomponenten

Besonders gut eignen sich die Block-Copolyester der Formel (I) als Emulgatoren für Wasser oder wäßrige Phasen in polaren Ölkomponenten. Als polare Ölkomponente werden dabei Öle verstanden, die im Molekül 1 bis 4 Estergruppen oder Ether-Sauerstoffatome enthalten. Eine bevorzugte Ausführung der Erfindung besteht daher darin, daß für die Wasser-in-Öl-Emulsion als Ölkomponenten ein- oder mehrwertige Ester von $C_8$-$C_{22}$-Fettsäuren oder von $C_8$-$C_{22}$-Fettalkoholen oder Ether von $C_8$-$C_{22}$-Fettalkoholen mit jeweils 16 bis 70 Kohlenstoffatomen verwendet werden. Bevorzugt sind die polaren Ölkomponenten bei 20°C flüssig. Solche für die Herstellung von Wasser-in-Öl-Emulsionen bevorzugten Ölkomponenten sind z.B. Fettsäureester wie z.B. Butylstearat, Cetyloleat, Isotridecylstearat, Oleyloleat, Jojobaöl, Ethylenglycoldiisostearat, Fettalkoholester wie z.B. Oleylacetat, Isotridecylbenzoat, Diisooctylsebazat und Triglyceridöle wie z.B. Olivenöl, Sonnenblumenöl, Sojaöl oder Palmöl.

Verfahren zur Herstellung der Emulsionen

Ein weiterer Gegenstand der Erfindung betrifft ein Verfahren zur Herstellung von Wasser-in-Öl-Emulsionen, welches sich dadurch auszeichnet, daß man der Ölphase 0,1 bis 10 Gew.-% Emulgatoren vom Typ der Block-Copolyester der allgemeinen Formel (I) zusetzt,

$$R^1CO\text{-}(OR^2CO)_y\text{-}(OC_2H_4)_x\text{-}O\text{-}(COR^2O)_z\text{-}COR^1 \qquad (I)$$

in der $R^1CO$ eine Acylgruppe mit 1 bis 22 Kohlenstoffatomen oder eine Hydroxyfettacylgruppe mit 16 bis 22 Kohlenstoffatomen, die Gruppe ($OR^2CO$) der Rest einer Hydroxyfettacylgruppe mit 16 bis 22 Kohlenstoffatomen, x eine Zahl von 5 bis 150, y und z Zahlen von 2 bis 75 sind, wobei die Gruppe $(OC_2H_4)_x$ wenigstens 10, aber weniger als 20 Gew.-% des Molekulargewichtes ausmacht. Die Emulgierung der dabei erhaltenen Emulsionskonzentrate mit Wasser oder einer wäßrigen Phase kann dann nach den üblichen mechanischen Emulgierverfahren erfolgen. Dabei wird die wäßrige Phase mit der Ölphase unter Anwendung von Scherenergie (Rühren, Schütteln, Ultraschall, Spalthomogenisierung, Rotor-Stator-Homongenisatoren) vermischt.

W/O-Emulsionen

Die erfindungsgemäßen Wasser-in-Öl-Emulsionen eignen sich für zahlreiche Anwendungen auf technischem und kosmetischem Gebiet. Mit Hilfe der erfindungsgemäß geeigneten Block-Copolyester der Formel (I) gelingt es beispielsweise, bis zu 80 Gew.-% Wasser oder wäßriger Lösungen in eine Ölphase einzuarbeiten. Ein weiterer Gegenstand der Erfindung betrifft daher Wasser-in-Öl-Emulsionen mit einem Gehalt von 1 bis 80 Gew.-% innerer wäßriger Phase und 20 - 99 Gew.-% Ölphase, wobei die Ölphase 0,1 bis 10 Gew.-% eines Stabilisators enthält, welche sich dadurch auszeichnen, daß sie als Stabilisatoren Block-Copolyester der allgemeinen Formel (I) enthalten,

$$R^1CO\text{-}(OR^2CO)_y\text{-}(OC_2H_4)_x\text{-}O\text{-}(COR^2O)_zCOR^1 \qquad (I)$$

in der $R^1CO$ eine Acylgruppe mit 1 bis 22 Kohlenstoffatomen oder eine Hydroxyfettacylgruppe mit 16 bis 22 Kohlenstoffatomen, die Gruppe ($OR^2CO$) der Rest einer Hydroxyfettacylgruppe mit 16 bis 22 Kohlenstoffatomen, x eine Zahl von 5 bis 150, y und z Zahlen von 2 bis 75 sind, wobei die Gruppe $(OC_2H_4)_x$ wenigstens 10, aber weniger als 20 Gew.-% des Molekulargewichtes ausmacht. Insbesondere auf dem Anwendungsgebiet der kosmetischen und pharmazeutischen emulsionsförmigen Zubereitungen kommt solchen Emulsionen vom Typ Wasser-in-Öl auf Basis polarer Ölkomponenten, z.B. natürlicher Triglyceridöle und natürlicher und synthetischer Fettsäure- und Fettalkoholester eine große Bedeutung zu. Für diese Anwendungsgebiete ist der Gegenstand der vorliegenden Erfindung daher von besonderem Wert.

**Beispiele**

Es wurden Emulsionskonzentrate durch Auflösen von 0,1 Gew.-% bzw. 0,2 Gew.-% bzw. 0,5 Gew.-% der folgenden Block-Copolyester A bis F in verschiedenen Ölkomponenten durch Auflösen der Block-Copolyester in Öl bei 60°C hergestellt.

(A) 16,7 Gew.-% Polyethylenglycol MG:1000 + 83,3 Gew.-% Ricinolsäure
(B) 16,7 Gew.-% Polyethylenglycol MG:600 + 83,3 Gew.-% Ricinolsäure
(C) 16,7 Gew.-% Polyethylenglycol MG:1550 + 83,3 Gew.-% Ricinolsäure
(D) 16,7 Gew.-% Polyethylenglycol MG:3000 + 83,3 Gew.-% Ricinolsäure
(E) 11,8 Gew.-% Polyethylenglycol MG:1000 + 88,2 Gew.-% Ricinolsäure

Sodann wurden Emulsionen aus 25 Gew.-% der Emulsionskonzentrate und 75 Gew.-% Wasser dadurch hergestellt, daß die Emulsionskonzentrate und das Wasser gemischt und die Mischung auf 60°C erwärmt und im Heidolph-Reagenzglasschüttler 3 Minuten homogenisiert wurde. Stabile W/O-Emulsionen wurden erhalten mit den Emulsionskonzentraten aus:

- 0,1 - 0,5 Gew.-% Block-Copolyester A bis D in 99,5 - 99,9 Gew.-% Sonnenblumenöl
- 0,1 - 0,5 Gew.-% Block-Copolyester A bis D in 99,5 - 99,9 Gew.-% Di-n-Octylether
- 0,1 - 0,5 Gew.-% Block-Copolyester A bis D in Weißöl (Winog® 40)
- 0,1 - 1 Gew.-% Block-Copolyester E in Di-n-Octylether
- 0,5 - 1 Gew.-% Block-Copolyester E in Sonnenblumenöl.

**Patentansprüche**

1. Verwendung von Block-Copolyestern der allgemeinen Formel **(I)**,

$$R^1CO\text{-}(OR^2CO)_y\text{-}(OC_2H_4)_x\text{-}O\text{-}(COR^2O)_z\text{-}COR^1 \qquad (I)$$

in der $R^1CO$ eine Acylgruppe mit 1 bis 22 Kohlenstoffatomen oder eine Hydroxyfettacylgruppe mit 16 bis 22 Kohlenstoffatomen, die Gruppe $(OR^2CO)$ der Rest einer Hydroxyfettacylgruppe mit 16 bis 22 Kohlenstoffatomen, x eine Zahl von 5 bis 150, y und z Zahlen von 2 bis 75 sind, wobei die Gruppe $(OC_2H_4)_x$ wenigstens 10, aber weniger als 20 Gew.-% des Molekulargewichtes ausmacht, zur Stabilisierung von Wasser-in-Öl-Emulsionen.

2. Verwendung nach Anspruch 1, **dadurch gekennzeichnet, daß** man Block-Copolyester der Formel **(I)** einsetzt, in der die Gruppen $(OR^2CO)_y$ und $(COR^2O)_z$ für Reste der Ricinolsäure oder der hydrierten Ricinusölfettsäure mit einem mittleren Kondensationsgrad y und z von 5 bis 30 stehen.

3. Verwendung nach den Ansprüchen 1 und 2, **dadurch gekennzeichnet, daß** man Block-Copolyester der Formel **(I)** einsetzt, in der x für Zahlen von 10 bis 70 steht.

4. Verwendung nach den Ansprüchen 1 bis 3, **dadurch gekennzeichnet, daß** man als Ölkomponenten zur Herstellung der Wasser-in-Öl-Emulsionen ein- oder mehrwertige Ester von $C_8$-$C_{22}$-Fettsäuren oder $C_8$-$C_{22}$-Fettalkoholen oder Ether von $C_8$-$C_{22}$-Fettalkoholen mit jeweils 16 bis 70 Kohlenstoffatomen einsetzt.

5. Verfahren zur Herstellung von Wasser-in-Öl-Emulsionen, **dadurch gekennzeichnet, daß** man der Ölphase 0,1 bis 10 Gew.-% Emulgatoren vom Typ der Block-Copolyester der allgemeinen Formel **(I)** zusetzt,

$$R^1CO\text{-}(OR^2CO)_y\text{-}(OC_2H_4)_x\text{-}O\text{-}(COR^2O)_z\text{-}COR^1 \qquad (I)$$

in der $R^1CO$ eine Acylgruppe mit 1 bis 22 Kohlenstoffatomen oder eine Hydroxyfettacylgruppe mit 16 bis 22 Kohlenstoffatomen, die Gruppe $(OR^2CO)$ der Rest einer Hydroxyfettacylgruppe mit 16 bis 22 Kohlenstoffatomen, x eine Zahl von 5 bis 150, y und z Zahlen von 2 bis 75 sind, wobei die Gruppe $(OC_2H_4)_x$ wenigstens 10, aber weniger als 20 Gew.-% des Molekulargewichtes ausmacht.

6. Wasser-in-Öl-Emulsionen mit einem Gehalt von 1 bis 80 Gew.-% innerer wäßriger Phase und 20 bis 99 Gew.-% Ölphase, wobei die Ölphase 0,1 bis 10 Gew.-% eines Stabilisators enthält, **da durch gekennzeichnet,** daß als Stabilisatoren Block-Copolyester der allgemeinen Formel (I) enthalten sind,

$$R^1CO\text{-}(OR^2CO)_y\text{-}(OC_2H_4)_x\text{-}O\text{-}(COR^2O)_z\text{-}COR^1 \qquad (I)$$

in der $R^1CO$ eine Acylgruppe mit 1 bis 22 Kohlenstoffatomen oder eine Hydroxyfettacylgruppe mit 16 bis 22 Kohlenstoffatomen, die Gruppe $(OR^2CO)$ der Rest einer Hydroxyfettacylgruppe mit 16 bis 22 Kohlenstoffatomen, x eine Zahl von 5 bis 150, y und z Zahlen von 2 bis 75 sind, wobei die Gruppe $(OC_2H_4)_x$ wenigstens 10, aber weniger als 20 Gew.-% des Molekulargewichtes ausmacht.

**Claims**

1. The use of block copolyesters corresponding to general formula (I):

$$R^1CO\text{-}(OR^2CO)_y\text{-}(OC_2H_4)_x\text{-}O\text{-}(COR^2O)_z\text{-}COR^1 \qquad (I)$$

in which $R^1CO$ is an acyl group containing 1 to 22 carbon atoms or a hydroxyfatty acyl group containing 16 to 22 carbon atoms, $(OR^2CO)$ is the residue of a hydroxyfatty acyl group containing 16 to 22 carbon atoms, x is a number of 5 to 150 and y and z are numbers of 2 to 75, the group $(OC_2H_4)_x$ making up at least 10 but less than 20% by weight of the molecular weight,
for stabilizing water-in-oil emulsions.

2. The use claimed in claim 1, **characterized in that** block copolyesters or formula (I), in which the groups $(OR^2CO)_y$ and $(COR^2O)_z$ are residues of ricinoleic acid or of hydrogenated castor oil fatty acid with an average degree of condensation y and z of 5 to 30, are used.

3. The use claimed in claims 1 and 2, **characterized in that** block copolyesters of formula (I) in which x has a value of 10 to 70 are used.

4. The use claimed in claims 1 to 3, **characterized in that** monofunctional or polyfunctional esters of $C_{8\text{-}22}$ fatty acids or $C_{8\text{-}22}$ fatty alcohols or ethers of $C_{8\text{-}22}$ fatty alcohols containing 16 to 70 carbon atoms are used as oil components for the production of the water-in-oil emulsions.

5. A process for the production of water-in-oil emulsions, **characterized in that** block copolyesters corresponding to general formula (I):

$$R^1CO\text{-}(OR^2CO)_y\text{-}(OC_2H_4)_x\text{-}O\text{-}(COR^2O)_z\text{-}COR^1 \qquad (I)$$

in which $R^1CO$ is an acyl group containing 1 to 22 carbon atoms or a hydroxyfatty acyl group containing 16 to 22 carbon atoms, $(OR^2CO)$ is the residue of a hydroxyfatty acyl group containing 16 to 22 carbon atoms, x is a number of 5 to 150 and y and z are numbers of 2 to 75, the group $(OC_2H_4)_x$ making up at least 10 but less than 20% by weight of the molecular weight,
are added to the oil phase as emulsifiers in a quantity of 0.1 to 10% by weight.

6. Water-in-oil emulsions containing 1 to 80% by weight of inner aqueous phase and 20 to 99% by weight of oil phase, the oil phase containing 0.1 to 10% by weight of a stabilizer, **characterized in that** a block copolyester corresponding to general formula (I):

$$R^1CO\text{-}(OR^2CO)_y\text{-}(OC_2H_4)_x\text{-}O\text{-}(COR^2O)_z\text{-}COR^1 \qquad (I)$$

in which $R^1CO$ is an acyl group containing 1 to 22 carbon atoms or a hydroxyfatty acyl group containing 16 to 22 carbon atoms, $(OR^2CO)$ is the residue of a hydroxyfatty acyl group containing 16 to 22 carbon atoms, x is a number of 5 to 150 and y and z are numbers of 2 to 75, the group $(OC_2H_4)_x$ making up at least 10 but less than 20% by weight of the molecular weight,
is used as the stabilizer.

**Revendications**

1. Utilisation de copolyesters séquencés de formule générale (I)

$$R^1CO\text{-}(OR^2CO)_y\text{-}(OC_2H_4)_x\text{-}O\text{-}(COR^2O)_z\text{-}COR^1$$

dans laquelle $R^1CO$ est un groupe acyle ayant de 1 à 22 atomes de carbone ou un groupe hydroxyacyle gras ayant de 16 à 22 atomes de carbone, le groupe $(OR^2CO)$ est le reste d'un groupe hydroxyacyle gras ayant de 16 à 22 atomes de carbone, x est un nombre entier allant de 5 à 150, y et z sont des nombres allant de 2 à 75, le groupe $(OC_2H_4)_x$ représentant au moins 10, mais moins de 20 % en poids de la masse moléculaire, pour la stabilisation d'émulsions eau-dans-huile.

2. Utilisation selon la revendication 1,
   **caractérisée en ce qu'**
   on utilise des copolyesters séquencés de formule (I) dans lesquels les groupes $(OR^2CO)_y$ et $(COR^2O)_z$ représentent des restes de l'acide ricinoléique ou de l'acide gras d'huile de ricin hydrogéné, ayant un degré moyen de condensation y et z de 5 à 30.

3. Utilisation selon les revendications 1 et 2,
   **caractérisée en ce qu'**
   on utilise des copolyesters séquences de formule (I), dans lesquels x représente les nombres 10 à 70.

4. Utilisation selon les revendications 1 et 3,
   **caractérisée en ce qu'**
   on utilise comme composant huileux, pour la préparation des émulsions eau-dans-huile, des esters mono- ou plurivalents d'acides gras en $C_8$-$C_{22}$ ou d'alcools gras en $C_8$-$C_{22}$ ou d'éthers d'alcools gras en $C_8$-$C_{22}$ ayant chacun de 16 à 70 atomes de carbone.

5. Procédé pour la préparation d'émulsions eau-dans-huile,
   **caractérisé en ce qu'**
   on ajoute à la phase huileuse 0,1 à 10 % en poids d'émulsifiants du type des copolyesters séquencés de formule générale (I)

$$R^1CO\text{-}(OR^2CO)_y\text{-}(OC_2H_4)_x\text{-}O\text{-}(COR^2O)_z\text{-}COR^1$$

dans laquelle $R^1CO$ est un groupe acyle ayant de 1 à 22 atomes de carbone ou un groupe hydroxyacyle gras ayant de 16 à 22 atomes de carbone, le groupe $(OR^2CO)$ est le reste d'un groupe hydroxyacyle gras ayant de 16 à 22 atomes de carbone, x est un nombre entier allant de 5 à 150, y et z sont des nombres allant de 2 à 75, le groupe $(OC_2H_4)_x$ représentant au moins 10, mais moins de 20 % en poids de la masse moléculaire.

6. Emulsions eau-dans-huile ayant une teneur en phase aqueuse interne de 1 à 80 % en poids et en phase huileuse de 20 à 99 % en poids, la phase huileuse contenant 0,1 à 10 % en poids d'un stabilisant,
   **caractérisées en ce que**,
   comme stabilisants, sont contenus des copolyesters de formule générale (I)

$$R^1CO\text{-}(OR^2CO)_y\text{-}(OC_2H_4)_x\text{-}O\text{-}(COR^2O)_z\text{-}COR^1$$

dans laquelle $R^1CO$ est un groupe acyle ayant de 1 à 22 atomes de carbone ou un groupe hydroxyacyle gras ayant de 16 à 22 atomes de carbone, le groupe $(OR^2CO)$ est le reste d'un groupe hydroxyacyle gras ayant de 16 à 22 atomes de carbone, x est un nombre entier allant de 5 à 150, y et z sont des nombres allant de 2 à 75, le groupe $(OC_2H_4)_x$ représentant au moins 10, mais moins de 20 % en poids de la masse moléculaire.